# NEW EUROPEAN PATENT SPECIFICATION

(11) **EP 0 389 570 B2**
(45) Date of publication and mention of the opposition decision: **19.07.2000**
(45) Mention of the grant of the patent: 15.02.1995
(21) Application number: 89903532.3
(22) Date of filing: 30.11.1988
(51) Int. Cl.: A61K 38/00

(54) **HIGH INTEGRITY LIPOSOMES AND METHOD OF PRERATION AND USE**
LIPOSOME HOHER STABILITÄT UND VERFAHREN ZUR HERSTELLUNG UND VERWENDUNG
LIPOSOMES A DEGRE DE STABILITE ELEVE ET PROCEDE DE PREPARATION ET D'UTILISATION DE TELS LIPOSOMES

(30) Priority: 04.12.1987 US 128974
(43) Date of publication of application: 03.10.1990
(73) Proprietor: THE LIPOSOME COMPANY, INC., Princeton, NJ 08540 (US)
(72) Inventor: WEINER, Alan, L., Lawrenceville, NJ 08648 (US); ESTIS, Leonard, F., Upton, MA 01568 (US); JANOFF, Andrew, S., Yardley, PA 19067 (US)
(74) Representative: Weiss, Wolfgang, Dipl.-Chem. Dr.
(86) International application number: US8804270
(87) International publication number: WO8905151

(56) References cited:
- EP-A- 0 172 007
- EP-A- 0 177 223
- EP-A- 0 220 797
- EP-A- 0 240 346
- WO-A-86/06959
- WO-A-88/01864
- US-A- 4 310 505
- US-A- 4 416 872
- US-A- 4 565 696
- US-A- 4 578 269
- US-A- 4 603 044
- US-A- 4 722 842
- US-A- 4 743 583
- US-A- 4 752 425
- US-A- 4 761 288
- US-A- 4 762 720
- Merck Index, 11th edition, 1989, page 854, "lecithin".
- DAVIS, D. et al.: IMMUNOLOGY, vol. 61, no. 2, 1987, pp. 229-234
- GREGORIADIS, G. et al.: VACCINE, vol. 5, 1987, pp. 145-151
- KIRBY, C.J. et al.: LIPOSOME TECHNOLOGY, vol. 1, no. 2, 1987, ed. G. Gregoriadis, pp. 19-27
- SENIOR, J. et. al.: FEBS LETTERS, vol. 145, no. 1, 1982, pp. 109-114
- SZOKA, F. et al.: PROC. NATL. ACAD. SCI., vol. 75, 1987, pp. 4194-4198
- HUANG, L. et al.: BIOCHEMISTRY, vol. 18, 1979, pp. 1702-1707
- MARCIA, R. et al.: ANALYTICAL BIOCHEMISTRY, vol. 94, 1979, pp. 302-307
- MERCK INDEX, 9th ed., 1976, Nos. 1633(pp.208) and 5287 (pp.711-712)

## Description

### Field of Invention

This invention relates to a high integrity liposome adapted for parenteral administration to an animal, including a human, and method for their manufacture. The liposome of the invention comprises a) at least one fully hydrogenated phosphatidylcholine stabile lipid which is not dicetylphosphate; b) a lipid diluent selected from cholesterol and alpha-tocopherol; and c) a therapeutically effective amount of at least one peptide-like therapeutic agent associated with said liposome, wherein the therapeutic agent is selected from antigens, hormones, immunomodulators, glycosylated carrier proteins and galactosylated carrier proteins. Such liposome is particularly useful in the extended release of peptide therapeutic agents as well as serving to protect said agents from degradation in the physiological environment.

### Background of the Invention

Peptide therapeutic agents are well known and of increasing use in the pharmaceutical arts. Hormones, immunomodulators, and a host of newly discovered peptide and peptide-like compounds are presently being administered to animals, including humans, in therapeutic regimens.

Consistent drawbacks to the parenteral administration of such peptide compounds have been the rapidity of breakdown or denaturation (loss of "native state configuration") of such compounds in the physiological environment and the difficulty of obtaining extended therapeutically effective dosage levels of such agents. Infusion pumps have been employed for chronic administration of therapeutic agents as well as wax or oil implants in an effort to both prolong the presence of peptide-like therapeutic agents and preserve the integrity of such agents.

Furthermore, in the particular case in which the peptide-like therapeutic agent (which will be understood to include a protein or hapten) is to function as an immunogen, the peptide-like agent should (with particular reference to each epitope of the peptide-like agent), ideally maintain native state configuration for an extended period of time and additionally be presented a fashion suitable for triggering an immunogenic response in the challenged animal.

U.S, Patent 4,310,505 discloses lipid vesicles comprising a lipid bilayer which includes analogs of cell-surface receptors and an effective amount of physiologically compatible radioactive tracer, cytotoxic or therapeutic agent as a part of the vesicles. The vesicles of this invention can be administered to the human host and have been found to release the contents of the vesicles in a predetermined manner, i.e. controlled release.

### Summary of the Invention

This invention includes a high integrity liposome adapted for parenteral administration to an animal, said liposome comprising a) at least one fully hydrogenated phosphatidylcholine stable lipid which is not dicetylphosphate (DCP) b) a lipid diluent selected from cholesterol and alphatocopherol; and c) a therapeutically effective amount of at least one peptide-like therapeutic agent associated with said liposome, wherein the therapeutic agent is selected from antigens, hormones, immunomodulators, glycosylated carrier proteins and galactosylated carrier proteins, wherein said therapeutical agent is not a tetanus toxoid and wherein said liposome does not contain stearylamine, 6-(5-cholesten-3β-yloxy)hexyl 1-thio-β-L-fucopyranoside, 6-(5-cholesten-3β-yloxy)hexyl 1-thio-β-D-galactopyranoside, 6-(5-cholesten-3β-yloxy)hexyl 1-thio-α-D-mannopyranoside, 6-(5-cholesten-3-yloxy)hexyl 2-acetamido-2-deoxy-1-thio-β-D-galactopyranoside, 6-(5-colesten-3β-yloxy)hexyl 6-amino-6-deoxy-1-thio-β-D-galactopyranoside, or 6-(5-cholesten-3β-yloxy)hexyl 6-amino-6-deoxy-1-thio-α-D-mannopyranoside. In some embodiments the stabile lipid is hydrogenated phosphatidylcholine or distearoyl phosphatidylcholine. In particular embodiments the therapeutic agent comprises an antigen. This invention further comprises the liposomes wherein the liposome further comprises a lipid diluent, such as cholesterol. In some embodiments containing cholesterol the cholesterol is present in a lipid:cholesterol molar ratio of from about 4:1 to about 1:1 (molar weight basis). In one embodiment distearoyl phosphatidycholine and cholesterol are used in about a 7:3 mole% ratio of phospholipid to cholesterol.

The peptide-like therapeutic agent in the liposomes of this invention include a hormone, an immunomodulator, glycosylated carrier protein, or galactosylated carrier protein, with particular reference to the peptide-like therapeutic agent being galactose-albumin, or the hormone being a somatotropin or calcitonin including analogues or deravatives thereof. In embodiments wherein the therapeutic agent is an immunomodulator, a particular example is an interleukin, such as IL2.

More particularly, the present invention provides a therapeutic pharmaceutical composition as claimed in claims 1 to 7.
The composition of this invention is suitable for treating an animal by administering to the animal a therapeutically effective dose of peptide-like therapeutic agent encapsulated in high integrity liposomes. The administration can be intramuscular, parenteral, intra-arterial, subcutaneous, intravenous, intraperitoneal which include the peptide-like therapeutic agent being a hormone or immunomodulator.

The composition for treating animals also includes the peptide-like therapeutic agent being a hormone, an immunomodulator or glycosylated carrier protein, or galactosylated carrier protein, such as wherein the peptide-like therapeutic agent are the hormones bovine somatotropin or calcitonin or wherein the peptide-like therapeutic agent is the immunomodulator IL2 (including analogues and deravatives of the foregoing).

The protein-like therapeutic agent of composition for treating animals can be a growth promotant, and the animal to be treated can be a pig, a chicken, a salmon a cow or a human.

In a particular aspect, this invention provides a method of increasing milk production in dairy animals by administering to the animals a therapeutically effective amount of somatotropin in high integrity liposomes. In one aspect the dairy animal is a cow and the somatotropin is bovine somatotropin or analogues or deravatives thereof.

### Brief Description of the Drawings

Figure 1 compares in situ retention of high integrity liposomes and liposomes of unhydrogenated lipid.
Figure 2 depicts weight gain by hypophysectomized rats administered BSTH in high integrity liposomes.

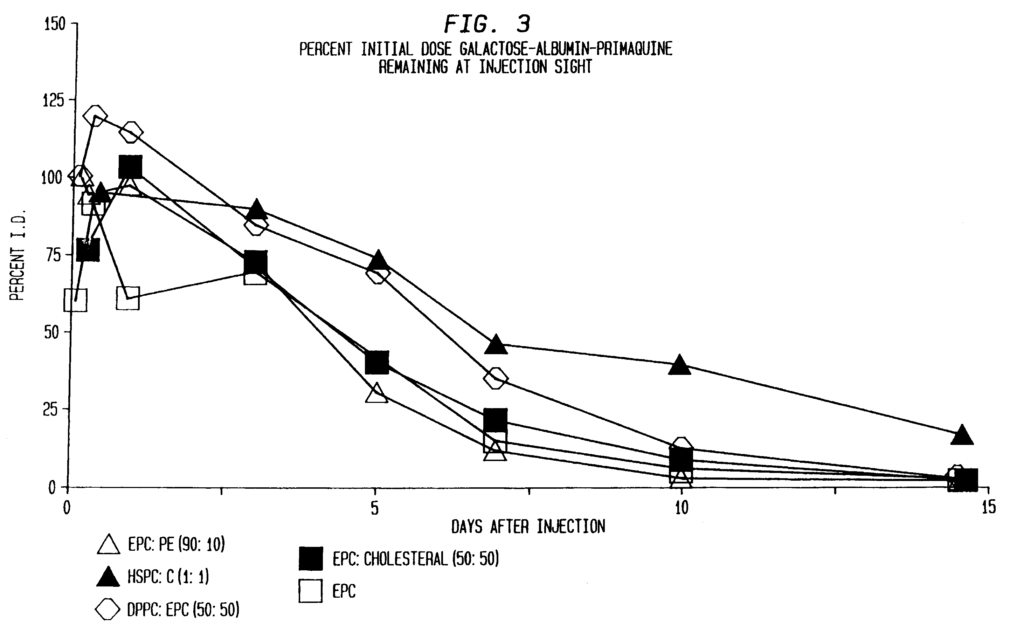

Figure 3. discloses in situ retention of dosage at injection site.

### Detailed Description of the Invention

Liposomes are completely closed lipid bilayer membranes containing an entrapped aqueous volume. Liposomes may be unilamellar vesicles (possessing a single bilayer membrane ) or multilamellar vesicles (onion-like structures characterized by multiple membrane bilayers, each separated from the next by an aqueous layer). The bilayer is composed of two lipid monolayers having a hydrophobic "tail" region and a hydrophilic "head" region. The structure of the membrane bilayer is such that the hydrophobic (nonpolar) "tails" of the lipid monolayers orient toward the center of the bilayer while the hydrophilic "head" orient towards the aqueous phase.

The original liposome preparation of Bangham, et al. (J. Mol. Biol., 1965, 12:238-252) involves suspending phospholipids in an organic solvent which is then evaporated to dryness leaving a phospholipid film on the reaction vessel. Next, an appropriate amount of aqueous phase is added, the mixture is allowed to "swell," and the resulting liposomes which consist of multilamellar vesicles (MLVs) are dispersed by mechanical means. This technique provides the basis for the development of the small sonicated unilamellar vesicles described by Papahadjopoulos et al. (Biochim. Biophys. Acta., 1968, 135:624-638), and large unilamellar vesicles.

Unilamellar vesicles may be produced using an extrusion apparatus by a method described in Cullis et al., PCT Application No. WO 87/00238, published January 16, 1986, entitled "Extrusion Technique for Producing Unilamellar Vesicles". Vesicles made by this technique, called LUVETS, are extruded under pressure through a membrane filter.

Another class of liposomes are those characterized as having substantially equal lamellar solute distribution. This class of liposomes is denominated as stable plurilamellar vesicles (SPLV) as defined in U.S. Patent No. 4,522,803 to Lenk, et al., monophasic vesicles as described in U.S. Patent No. 4558,579 to Fountain, et al. and frozen and thawed multilamellar vesicles (FATMLV) wherein the vesicles are exposed to at least one freeze and thaw cycle; this procedure is described in Bally et al., PCT Publication No. 87/00043, January 15, 1987, entitled "Multilamellar Liposomes Having Improved Trapping Efficiencies". Honda et al. Japanese Patent Pub. No. 60-155109 describes hydrogenated liposomes requiring 5% fatty acid. The liposomes of this invention may be prepared with substantially less than 5% fatty acid or without the presence of fatty acid at all.

A wide variety of lipids both saturated and unsaturated can be used in forming liposomes (See Iga et al., European patent application, Publication number 0 240 346 A3, published on October 7, 1987, entitled "Method of Producing Liposome".
Geho et al., US patent, patent number 4 603 044, dated July 29, 1986, entitled "Hepatocyte Directed Vesicle Delivery System", describes a lipid composition including distearoyl lecithin (DSL), dicetylphosphate (DCP) and cholesterol. The present invention does not use DCP.
Martin et al., PCT publication n° WO 88/01864, published on March 3, 1988, entitled "High Concentration Liposome Processing Method" use partially hydrogenated soy phosphatidylcholine (PHSPC).
Mezei, European patent application n° 0 177 233 A3, dated April 9, 1986, entitled "Pharmaceutical Multiphase Composition" discloses compositions of liposomes which are all for topical formulations.
Felgner et al., European patent application n° 0 172 007, published on February 19, 1986, entitled "Stable Liposomes with aqueous-soluble medicaments and methods for their preparation" describes the combination of a stabile lipid and a lipid diluent selected from cholesterol and alphatocopherol. This reference includes DPPG as an essential component.
Matumoto et al., European patent application n° 0 220 797 A2, published on May 6, 1987, entitled "Process for the Preparation of Liposome" shows the use of a variety of phospholipids, saturated and unsaturated, hydrogenated and unhydrogenated with a detergent to form a liposome.
Mihalko et al., PCT publication n° WO 86/06959, published on May 21, 1986, entitled "Liposome Inhalation Method and System", describes pharmaceutical compositions delivered to the respiratory tract by inhalation. Various lipids are discussed for use in this composition, both stabile and non-stabile lipids.

For use in the present invention, cholesterol and alpha-tocopherol are used to form liposomes; see specifically Janoff et al., PCT Publication No. WO 85/04578, published October 24, 1985, entitled "Steroidal Liposomes." Mayhew et al., PCT Publication No. WO 85/00968, published March 14, 1985, described a method for reducing the toxicity of drugs by encapsulating them in liposomes comprising alpha-tocopherol and certain derivatives thereof. Also, a variety of tocopherols and their water soluble derivatives have been used to form liposomes, see Janoff et al., PCT Publication No. WO 87/02219, published April 23, 1987, entitled "Alpha Tocopherol-Based Vesicles".

The term lipid as used herein shall mean any suitable material resulting in a bilayer such that a hydrophobic portion of the lipid material orients toward the interior of the bilayer while a hydrophilic portion orients toward the aqueous phase. Lipids further include highly hydrophobic compounds such as triglycerides, sterols such as cholesterol which can be incorporated into the bilayer. The term lipid does not include fatty acids. Specific lipids are phospholipids such as phosphatidylcholine (PC), phosphatidylethanolamine (PE), distearoyl phosphatidylcholine (DSPC), phosphatidylserine (PS), phosphatidylglycerol (PG), phosphatidic acid (PA), phosphatidylinositol (PI), sphingomyelin (SPM), and the like, alone or in combination. The phospholipids can be synthetic or derived from natural sources such as egg or soy. Some synthetic phospholipids are dymyristoylphosphatidylcholine (DMPC) and dimyristoylphosphatidylglycerol (DMPG). Liposomes can also contain other steroid components such as polyethylene glycol derivatives of cholesterol (PEG-cholesterols), coprostanol, cholestanol, or cholestane, and combinations of PC and cholesterol. Liposomes may also contain glycolipids.

"Stabile lipid" shall be understood to be lipids which are resistant to oxidative catabolism initiated by changes in pH, temperature, oxygen free radicals (e.g., such as those produced by infiltrating immune cells during inflamatory reaction) or other stress of the physiological environment.

It is to be understood that stabile is a property in the nature of a continuum whereby normal lipid rigidity is modified by a stabilizing process such as hydrogenation. When used in an environment of low pH such as the intragastric environment, excluded from the grouping of stabile lipids are lipids such as cholesterol hemisuccinate or tocopherol hemisuccinate which are deconstructed at the physiologic pH ranges found in animal gastrointestinal systems. In such low pH applications these are excluded without regard to rigidity. Thus a stabile lipid is first a lipid resistant to oxidative catabolism initiated by changes in pH, as well as resistant to temperature, oxygen free radicals or other stress of the physiological environment and second not deconstructed at common physiologic pH ranges presented in the in vivo environment of use. Stabile lipids, when organized into liposomes, will maintain structural integrity for an extended period of time, particularly as compared to other liposomes.

The term peptide-like will be understood to mean short chain peptides as well as well as proteins. A preferred class of peptides is immunomodulators such as interleukins, colony stimulating factors and interferons. An additionally preferred class of proteins are antigens such as is used in vaccines.

The term native state configuration will be understood to mean that organization of a moiety having at least one epitope, such as a peptide, as it is configured when present in vivo, to be distinguished from non-native state configuration (denatured) wherein the moiety is altered as to bioactivity or immuno-reactivity from that of the in vivo organization.

The term epitope will be understood to mean the smallest part of an antigen moiety recognizable by the combining site of an immunoglobulin.

When using a protein merely as a proteinic macromolecule carrier of a therapeutic agent, maintenance of the native state of such protein is of lesser importance, so long as the carrying function is not substantially compromised. For example, albumin has been reported as used as a "carrier protein" for therapeutic agents, particularly as linked to galactose ("galactose-albumin") or glucose ("glycosylated-albumin"), thus facilitating hepatic uptake when presented in liposomal form (e.g., U.S. Patent Ser. No. 4,376,765). As used herein "galactose-albumin" (and similarly glycose-albumin) refers to the moiety of galactose (or glucose) joined to albumin. Such moiety -- glucose or galactose/carrier protein -- is useful in targeting a therapeutic agent at the liver. The moiety is preferentially taken up by the liver, and by covalently joining a therapeutic agent to the carrier protein the therapeutic agent is similarly taken up by the liver. By way of example, the therapeutic agents doxorubicin, daunarubicin, or primaquine may be joined to the carrier protein to then be concentrated in the liver, thus localizing the therapeutic action of the agent (here, anticancer and antiparisitic).

In some embodiments, liposomes of this invention, in association with peptides, function as adjuvants.

"Labile" therapeutic agents as used herein refers to the propensity for destruction or denaturation of the therapeutic agent in an animal by reactions other than the intended therapeutic reactions.

Preferred liposomes of this invention were prepared from fully hydrogenated soy phosphatidylcholine and cholesterol in ratios of from about 4:1 to about 1:1 (phosphatidylcholine:cholesterol mole ratio). These liposomes were administered to animals and the animals then tested for retention of administered material for periods up to about 2 weeks post administration. Administered material was found to be present at the site of administration for up to two weeks at a level of about 20% when administered via intramuscular or subcutaneous administration. The extended retention at injection site has facilitated extended release of peptides over this period, while maintaining much of the integrity of the administered material, particularly peptides. Other preferred liposomes are of DSPC.

The administration profile that is therapeutically indicated will be influenced by many considerations including the increasing ease of handling the stabile lipids, the therapeutic agent to be incorporated into the liposome, the site of administration of the liposome preparation, and the nature and condition of the animal being treated.

The term extended elaboration as used herein is understood to mean the release of therapeutic agents from liposomal encapsulation over a period in excess of about 24 hours and in some embodiments as long as about 2 to 3 weeks.

Structural integrity of liposomes as used herein shall mean the substantial maintenance of the pharmaceutical activity of the encapsulated substance during a period of extended elaboration. This structural integrity is presumed to arise from the persistence of the bilayer arrangement of the lipid material comprising the liposomes and the concomitant substantial maintenance of an entrapped aqueous phase for the period of extended elaboration. Structural integrity may be imparted by forming liposomes from combinations of lipids comprising sufficient stabile lipid to maintain the required structure when challenged by the physiological conditions present in the subject animal. In particular embodiments the high integrity liposome of this invention structural integrity will be maintained even if stabile lipid is admixed with a diluent lipid or secondary lipid which is not stabile. In the practice with this invention liposomes of sufficient structural integrity for the intended use may be designed by varying the rigidity of lipid membrane constituents or by varying the proportion in which stabile lipid is admixed with a diluent or secondary lipid.

In the instant invention modifications of these above noted procedures for making liposomes are required due to the high rigidity of stabile lipids. In the most stabile lipids -- such as fully hydrogenated phosphatidylcholine -- if a lipid film in the preparation process it may be solubilized in organic solvent at an elevated temperature often about 50-60°C. This increases the flexibility of the lipid and permits formulation of liposomes.

Stabile lipids of greater flexibility may be prepared by a variety of methods. Hydrogenation of lipid to less than full hydrogenation produces a lipid of increased but less than maximum rigidity. Additionally, fully or partially hydrogenated lipids or other stabile lipids may be admixed with unsaturated flexible lipids. Cholesterol as an admixing material is unique in that it tends to make stabile lipids more flexible while conversely having the property of rendering flexible lipids more rigid. Cholesterol is a preferred admixing lipid to increase the flexibility of the stabile lipid component of the liposomes of this invention. Additionally, alpha-tocopherol may function as an admixing lipid.

High integrity liposomes of this invention may be incorporated with therapeutic agents by the methods well known in the art such as those of U.S. Patent Nos. 4,522,803 and 4,588,578 and the Mayer article, supra. Particular embodiments in the practice of this invention are the incorporation of peptide therapeutic agents such as growth hormone or growth hormone releasing factor.

Particular benefits of the liposomes of this invention are the enhancement of drug entrapment upon formation of the liposomes, and the enhancement of amount of drug per amount of lipid (loading).

The entrapment of up to about 70% of available peptide-like therapeutic agents are obtainable by the instant method. It is important to note that peptide entrapment levels are dependent on the specific peptide-like therapeutic agents being entrapped.

Liposomes entrap an aqueous medium which is enclosed by the lipid bilayers. The aqueous medium can be for example, water or water containing a dissolved salt or buffer. Examples of such salts or buffers can be sodium chloride and phosphate buffered saline (PBS). Other buffers include borate, citrate, Tris-HCl(Tris-(hydroxymethyl)-aminomethane hydrochloride), and HEPES (N-2-hydroxyethyl piperazine-N¹-2-ethane sulfonic acid). Buffers may be in the pH range of between about 2.0 and about 14.0. In particular embodiments, the preparations are hydrated with HEPES buffer (150 mM NaCl, 20mM HEPES), pH 7.0, borate buffer (100 mM Na₂HCO₃, 50 mM H₃BO₃), pH 8.5, or citrate buffer (150 MM Na-citrate), pH 8.5, or 0.01M sodium carbonate buffer (pH 9-11).

The loading of liposomes (i.e.,lipid:peptide) is also enhanced by the use of stabile lipids in the practice of this invention. For example, when liposomes entrapping BSTH were formed in the presence of lipid:BSTH at 2:1 the liposomes thus formed were 6.1:1 lipid:peptide as opposed to 16.2:1 for similar liposomes formed from unhydrogenated lipids. In another instance liposomes prepared from lipid:galactose-albumin (1.8:1 feed) yielded a lipid:galactose-albumin liposome of 4.3:1 as opposed to 7.1:1 for unhydrogenated liposomes at a similar lipid:galactose-albumin feed ratio.

In another example, DSPC liposomes, mixed with cholesterol (preferably 7:3 mole% ratio of phospholipid to cholesterol), were formed entrapping calcitonin. Calcitonin is available in many forms and analogues and derivatives of calcitonin are being developed or are now available and all are understood to be included in the term calcitonin. DSPC is obtainable from Avanti Polar Lipids (Birmingham, Ala.). The high integrity liposomes of this invention extended the presence of detectable calcitonin from about 1 hour for free calcitonin to about 3 to 7 days.

In a liposome-drug delivery system, the therapeutic agent is encapsulated in the liposome (either in the lipid or aqueous phase), and then administered to the subject being treated. For example, see Rahman et al., U.S Patent No. 3,993,754; Sears, U.S Patent No. 4,145,410; Papahadjopoulos et al., U.S Patent No. 4,235,871; Schneider, U.S. Patent No. 4,224,179, Lenk, et al., U.S Patent No. 4,522,803, and Fountain et al., U.S. Patent No. 4,588,578.

The liposomes may be dehydrated, thereby enabling storage for extended periods of time until use. Standard freeze-drying equipment or equivalent apparatus may be used to dehydrate the liposomes. Liposomes may also be dehydrated simply by placing them under reduced pressure. Alternatively, the liposomes and their surrounding medium can be frozen in liquid nitrogen prior to dehydration. Dehydration with prior freezing may be performed in the presence of one or more protective sugars in the preparation, according to the process of Janoff et al., U.S. Patent Application Serial No. 759,419, filed July 26, 1985, entitled "Dehydrated Liposomes". Examples of protective sugars that may be used include, but are not limited to, trehalose, maltose, sucrose, glucose, lactose and dextran. When the dehydrated liposomes are to be used, rehydration is accomplished by methods which include simply adding an aqueous solution, e.g., distilled water, to the liposomes and allowing them to rehydrate.

The therapeutic agents of this invention can be administered associated with liposomes, in admixture with a pharmaceutically-acceptable carrier selected with regard to the intended route of administration and standard pharmaceutical practice.

Therapeutic agents are peptide-like therapeutic agents which include the peptide-like agents such as galactose-albumin carriers, immunomodulators (e.g., interleukins) and hormones (e.g., somatotropins). Particular therapeutic agents referred to are understood to include analogues and derivatives of such agents also including biologically active fragments unless specifically indicated to not so include.

Pharmaceutical dosage forms of the present inventions could be comprised of liposomes and any suitable pharmaceutical carrier. A preferred class of carrier would be aqueous including both distilled water and isotonic saline. Administration of high integrity liposomes might be accomplished by any usual route with particular reference to subcutaneous and intramuscular administration. Parenteral administration as used herein refers to intra muscular, intravenous, intra-articular, and intra-ocular administration. However, dosages adapted to parenteral administration might be used in a variety of administration methods.

Dosages for therapeutic agents associated with liposomes will often be about that of the therapeutic agent alone; dosages will be set by the prescribing medical professional considering many factors including the age, weight and condition of the patient. The ratio of therapeutic agent to carrier will naturally depend on the chemical nature, solubility, trapping efficiency, and stability of the therapeutic agent, as well as the dosage contemplated. For parenteral administration or injection via such routes as intravenous, intraperitoneal, intramuscular, subcutaneous, or intra-mammary route, sterile solutions of the liposome composition are prepared. For intravenous use, the total concentration of solutes should be controlled to render the preparation isotonic.

In the application of this invention to animal husbandry those skilled in the art will understand the use of high integrity liposomes in the administration of a number of therapeutic agents including growth promotants (e.g., growth hormome and growth releasing factor) as well as lactation promoting agents such as BSTH. Administration of such agents in therapeutically effective amounts can increase productivity.

Dosage and administration of therapeutic agents, in this invention, presumes therapeutically effective amounts. Therapeutically effective amounts of therapeutic agents as used herein will mean that amount of therapeutic agent that produces therapeutic action. This amount will be understood to vary with the particular agent or analog or derivative thereof, the condition being treated, the site, manner and duration of administration and other considerations known to those skilled in the art.

In the application of this invention to the vaccine art, the necessary dosage will be understood to be an immunogenic dosage. It will be understood that an immunogenic amount of an antigen protein is that amount which will stimulate the response cells of a subject animal to produce immunoglobulins against the antigen. This amount will vary with the potency of adjuvant, with the mode of administration and with the type and condition of animal but is easily determined by any of the well known tests for immunoglobulins with an increase in immunoglobulin representing immunogenic response.

### Example 1

### Preparation of High Integrity Liposomes

A solution of 14g of bovine somatotrophic hormone (BSTH) in 140ml carbonate buffer, pH 10.9 was prepared. Then 21.1g of hydrogenated soy phosphatidylcholine (HSPC) in powdered form and 6.9g of cholesterol in powdered form were dissolved in 50ml chloroform and dried to powder by rotoevaporation. The lipid film thus formed was resuspended in 140ml diethyl ether and placed in a round bottom flask, and sonicated in a water bath at 47°C during addition of the BSTH in buffer. Sonication was continued until substantially all ether was evaporated. A stream of 20°C nitrogen was then applied to the resulting material until residual ether was removed.

The resulting material was then resuspended in 800ml of buffer at 47 - 50°C and the liposomes therein washed two times by repeated centrifugation at approximately 20,000 times gravity for 30 minutes. The washed liposomes were then resuspended to a final volume of 164ml.

The resulting high integrity liposome suspension contained 27.5mg BSTH/ml, 128.0mg HSPC/ml and 42.0mg cholesterol/ml.

### Example 2

### Increasing Milk Production

The liposome preparation of Example 1 was administered to cows at two 350mg doses per cow with the second dose two weeks after the original dose. This regimen produced a 14.9% increase in milk production as compared to untreated cows. Furthermore, as compared to cows treated with daily injection of 12.5mg free BSTH, two injections of BSTH in hydrogenated soy phosphatidylcholine liposomes was 28.8% as effective. Liposomes of unhydrogenated soy phosphatidylcholine, delivered in 3 weekly doses of 175mg BSTH over three weeks, were only 15.7% as effective as daily doses.

### Example 3

### Extended Elaboration/Retention of High Integrity Liposomes In Situ

### Preparation:

6.0g of BSTH was dissolved on 60.0ml of carbonate buffer pH 9.4. Powdered HSPC, 3.52g and powdered cholesterol, 1.73g were dissolved in 20ml chloroform in a 500ml round bottom flask. To this solution was added 1.2 x 10⁶dpm of ³H-dipalmitoyl phosphatidylcholine. The lipids were then dried by rotoevaporation, and resuspended in 75ml diethyl ether. The flask and contents were then placed in a 45°C water bath sonicator. The BSTH solution was then added to the diethyl solution while sonicating as the ether evaporated. After 15 minutes residual ether was removed by applying a stream of nitrogen to the contents of the flask. The contents of the flask was then resuspended in 150ml carbonate buffer and the resulting liposomes washed two times by centrifugation and brought up to a final volume of 34.0ml.

### Elaboration/Retention:

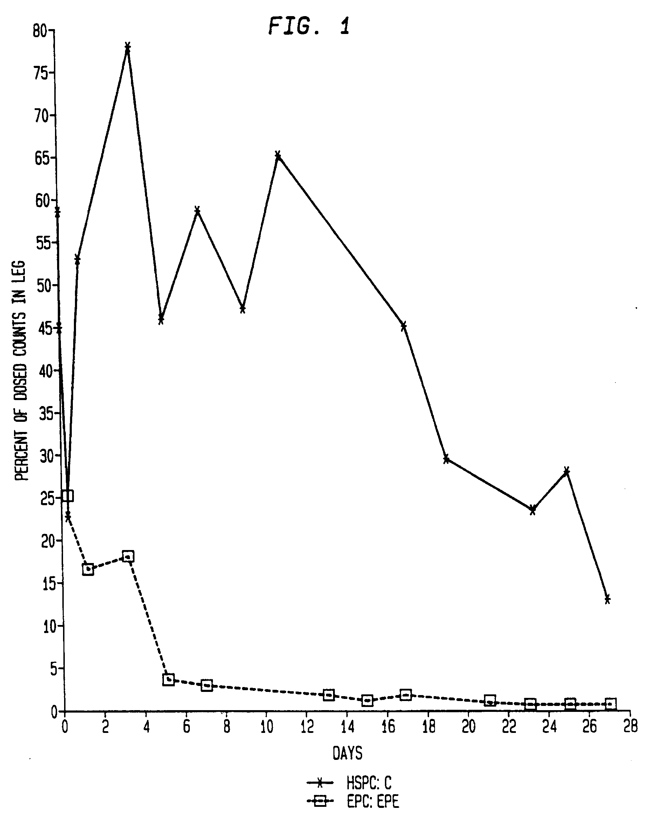

A 0.320ml dose of the liposome suspension was injected intramuscularly (leg) into each of 30 Swiss-Wistar mice. This corresponded to 9.77 x 10⁵dpm per animal. Three mice were sacrificed at each time point over a period of 27 days. The percentage of radioactivity remaining at the site of injection was compared to similar injection of unhydrogenated liposomes (egg phosphatidylcholine with egg phosphatidylethanolamine). Radioactivity in the mice receiving high integrity liposomes was still present after 27 days while the radioactivity of the unhydrogenated liposomes had almost entirely dissipated as is shown in Fig. 1.

### Example 4

### Extended Elaboration/Retention of High Integrity Liposomes In Situ

### Preparation:

0.75g of BSTH were dissolved in 15ml carbonate buffer, pH 10.9.

1.13g of HSPC and 0.37g of cholesterol are dissolved in 5ml chloroform. The mixture was dried under rotoevaporation as in Example 3, and the lipid resuspended in 20ml of diethyl ether, and placed in a round bottom flask.

The flask and contents were placed in a 47°C water bath sonicator, the sonicator turned on, and then the BSTH in the aqueous phase was added. Sonication was continued until the weight of the mixture was equal to the additive weights of the dried lipid film and the aqueous phase. A stream of nitrogen gas was then applied to remove any residual ether. The contents of the flask was then resuspended in 40ml of carbonate buffer at 47-50°C and the liposomes therein were washed two times by repeated centrifugation at approximately 20,000 times gravity for 30 minutes. The liposome suspension was then brought to a final volume of 11.1ml.

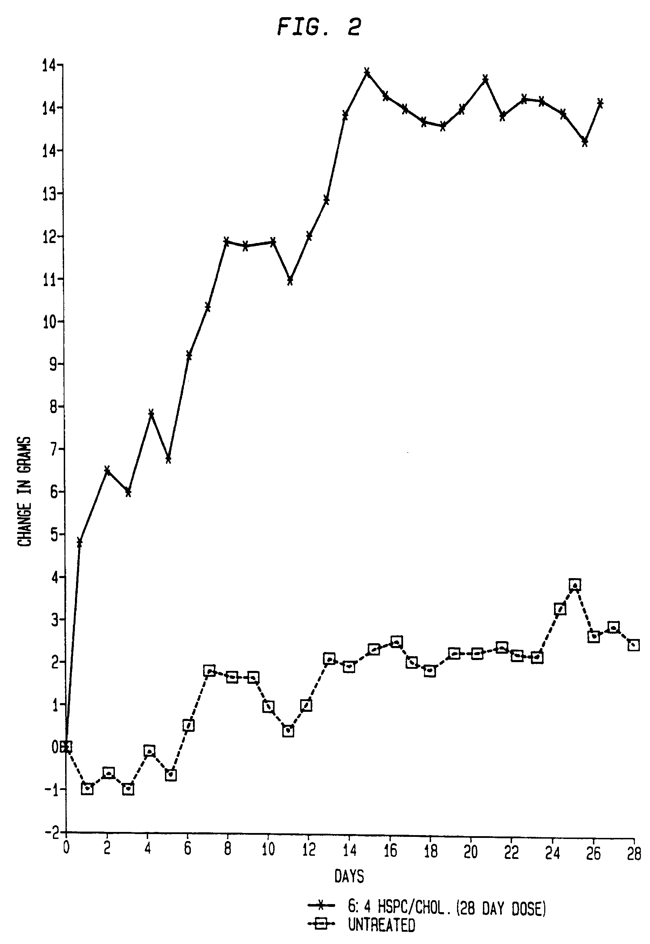

Fig 2 depicts the effect of these liposomes showing substantial weight gain over 28 days by hypophysectomized rats (75-85gm/rat)administered BSTH in the high integrity liposomes as compared to hypophysectomized rats administered single identical amounts of 80ug/day (2,400ug in total per animal) injected i.m. in the hind leg of BSTH in unhydrogenated liposomes.

### Example 5

### IL-2 Retention and Release

67.0mg HSPC and 33.0mg cholesterol were dried from chloroform by rotoevaporation in a round bottom flask. Then 5ml of anhydrous diethyl ether was added to the flask. To this was added 1mg of IL-2 in 1ml of 5mM ammonium acetate (pH5), 0.9% sodium chloride along with 6.0 x 10⁵ **DPM** of ³H-IL-2.

The resulting dispersion of aqueous solution in ether was sonicated in a water bath at 45°-50°C and simultaneously dried with a stream of nitrogen gas until no trace of ether was detectable by smelling. To the resulting dried liposome paste was added to 10ml of phosphate buffered saline. The mixture was vortexed vigorously to remove any material that had adhered to the flask wall. Aliquots of this mixture were taken for tritium counting.

The remaining mixture, a liposome suspension, was centrifuged for 20 minutes in a J-20 rotor (Beckman Instruments, Mountainview, CA.) at 10,000rpm. The supernatant was removed and an additional 10ml of phosphate buffered saline was used to resuspend the liposomes of the pellet.

Table 1 shows the substantial period (14 days) over which IL-2 remains at the injection site in mice receiving 2.7mg /kg in 10mg of high integrity liposomes.

Table 2 Shows that the retention was accompanied by persistence of available bioactive interleukin. Such persistence is seen to be quite pronounced as compared to free interleukin or to liposomal interleukin with other than high integrity liposomes.

### Example 6

### Galactose-Albumin-Primaquine

HSPC:Cholesterol liposomes were prepared according to the method of Example 1. Briefly, lipid in chloroform was dried as a thin film on the bottom of a round bottom flask by rotoevaporation. 5ml of diethyl ether were added to the flask and the lipid dislodged from the flask walls by swirling. In this example the material to be entrapped was human serum albumin conjugated to tritiated galactose ("galactose-albumin") and primaquine
("galactose-albumin-primaquine"). The galactose-albumin-primaquine in 0.3ml of aqueous solution was added to the 5ml of ether-lipid mixture.

Conjugate formation was effected by simultaneous sonication and drying of the mixture using a gentle stream of nitrogen. Sonication and drying were discontinued when no odor of ether could be detected. The conjugate material was in the form of a paste which was washed with 10-20ml of phosphate buffered saline (PBS). This was accomplished by adding PBS to the flask and vortexing vigorously to remove all residues from flask walls.

The resulting liposome suspension was then centrifuged at 4°C for 10-20 minutes at 10,000rpm (J-20 Centrifuge, JA-20 rotor, Beckman). The resulting supernatant was poured off and the pellet resuspended in fresh PBS, vortexed and centrifuged again under the same conditions two times to remove non-entrapped material.

193 mg of the resulting encapsulated containing 10⁷ disentegrations per minute material was injected intramuscularly into the hind leg of mice.

The retention of high integrity liposomes containing galactose-albumin can be seen from Table 3 and Table 4 to be at least 14 days. Table 3 discloses the retention at site of injection of radioactivity for up to 14 days when the radioactive material was encapsulated in high integrity liposomes, while, in contrast, free radioactive material was eliminated in a single day. Table 4 discloses that injected encapsulated material remaining at the site of injection retains bioactivity for en extended period as compared to the activity retention of unencapsulated material.

### Example 7

### Calcitonin-DSPC

8.27mg DSPC (1:1 initial L:D ratio) and 1.73mg cholesterol (7:3 mole% ratio DSPC: cholesterol), both in chloroform, were transferred to a 50 ml round bottom flask. The lipids were dried to a film under vacuum using rotoevaporation. The lipids were then solubilized in 0.500 ml methanol with heating for 1-2 mins. in a 60° water bath.

Ten milligrams of calcitonin (Mitsubishi Chemical Co., Japan MCI-536) was solubilized in 0.100 ml sodium acetate buffer. This solution was added to the solvent mixture.

The solvent was removed under vacuum, using rotoevaporation in a 60° water bath. Once the solvent was removed, the lipid/drug film was resuspended in 0.5 ml 60° sodium acetate buffer. The preparation was washed by addition of 0.5 ml of 60° sodium acetate buffer follwed by centrifugation at 12,100 xg for 10 minutes. The supernatant was decanted and the liposomal pellet resuspended with 1 ml of 60° sodium acetate buffer. The suspension was again centrifuged at 12,100 xg for 10 minutes and resuspended. The resuspended DSPC-cholesterol liposomes containing calcitonin were administered s.c. to mice to compare retention of the liposomal preparation to that of free calcitonin. Free calcitonin was undetectable after one hour in mice. The calcitonin of liposomes of the instant invention was at least about 70% present after one day from administration and persisted for about 3 to 7 days disclosing a substantial increase in retention time over free calcitonin.

**TABLE 1**

| I.M. SLOW RELEASE OF ³H LABELED PEPTIDES FROM HSPC/CHOL LIPOSOMES IN MICE | |
|---|---|
| Time After Injection | % of Dose Remaining at Injection Site |
| | Interleukin 2 |
| 1 Hr | 84.4 |
| 1 Day | 78.1 |
| 4 days | 64.9 |
| 7 Days | 69.4 |
| 10 Days | 58.9 |
| 14 Days | 28.1 |

**TABLE 2**

| RECOVERY OF "BIOACTIVE" PEPTIDE AT THE INJECTION SITE (IL₂) | | | |
|---|---|---|---|
| Time | % of Initial Bioactive IL₂ Remaining at Injection Site | | |
| | Free IL₂ | IL₂-EPC SPLV | IL₂-HSPC/CHOL SPLV |
| 1 Hr | 2.1 | 53.5 | 52 |
| 1 Day | ND | 15 | 44 |
| 4 Days | ND | 4.8 | 20 |
| 7 Days | ND | 1.3 | 11 |
| 10 Days | ND | ND | 18 |
| 14 Days | ND | ND | 14 |

**TABLE 3**

| I.M. SLOW RELEASE OF ³H LABELED PEPTIDES FROM HSPC/C LIPOSOMES IN MICE | | |
|---|---|---|
| Time After Injection | % OF DOSE REMAINING AT INJECTION SITE | |
| | HSPC Galactose-Albumin | Free Galactose-Albumin |
| 1 Hr | 109 | 55.7 |
| 7 Hrs | | 29.75 |
| 1 Day | 107 | 25.5 |
| 1.75 Days | | 11.23 |
| 2.75 Days | | 5.8 |
| 3 Days | 97 | |
| 5 Days | 75 | |
| 5.75 Days | | 1.8 |
| 7 Days | 59 | |
| 10 Days | 47 | |
| 14 Days | 23 | |

**TABLE 4**

| CHANGE IN FRAGMENTATION OF PEPTIDE FOLLOWING LIPOSOME ENCAPSULATION | | |
|---|---|---|
| Time | % OF PEPTIDE AT INJECTION SITE THAT IS FRAGMENTED | |
| | Free Galactose-Albumin-Primaquine | HSPC/C ENCAPSULATED Galactose-Albumin-Primaquine |
| 1 Hr | 16 | 1 |
| 7 Hrs | 87 | 1 |
| 2 Days | 100 | 1 |
| 3 Days | 100 | 7 |
| 5 Days | 100 | 15 |
| 7 Days | 100 | 21 |
| 10 Days | 100 | 17 |
| 14 Days | 100 | 16 |

## Claims

1. A therapeutic pharmaceutical composition suitable for parenteral administration which exhibits extended elaboration release characteristics upon parenteral administration in an animal, the composition comprising high-integrity liposomes which comprise a) at least one fully hydrogenated phosphatidylcholine stabile lipid which is not dicetylphosphate (DCP); b) a lipid diluent selected from the group consisting of cholesterol and alphatocopherol; and c) a therapeutically effective amount of at least one peptide-like therapeutic agent associated with said liposomes, wherein said therapeutic agent is selected from the group consisting of antigens, hormones, immunomodulators, glycosylated carrier proteins and galactosylated carrier proteins, wherein said therapeutical agent is not a tetanus toxoid and wherein said liposome does not contain stearylamine, 6-(5-cholesten-3β-yloxy)hexyl 1-thio-β-L-fucopyranoside, 6-(5-cholesten-3β-yloxy)hexyl 1-thio-β-D-galactopyranoside, 6-(5-cholesten-3β-yloxy)hexyl 1-thio-α-D-mannopyranoside, 6-(5-cholesten-3-yloxy)hexyl 2-acetamido-2-deoxy-1-thio-β-D-galactopyranoside, 6-(5-cholesten-3β-yloxy)hexyl 6-amino-6-deoxy-1-thio-β-D-galactopyranoside, or 6-(5-cholesten-3β-yloxy)hexyl 6-amino-6-deoxy-1-thio-α-D-mannopyranoside.

2. The composition of claim 1, wherein the stabile lipid is hydrogenated soy phosphatidylcholine or distearoyl phosphatidylcholine.

3. The composition of claim 1 or 2, wherein the lipid diluent is cholesterol in a lipid:cholesterol molar ratio of from 4:1 to 1:1.

4. The composition of any one of claims 1 to 3, wherein the peptide-like therapeutic agent is galactose-albumin, calcitonin, a somatotropin, an interleukin, or an analogue or derivative of any of these.

5. The composition of claim 4, wherein the peptide-like therapeutic agent is interleukin-2.

6. The composition of any one of claims 1 to 3, wherein the peptide-like therapeutic agent is a peptide, and the liposomes function as an adjuvant.

7. The composition of claim 5, wherein the peptide-like therapeutic agent is calcitonin, or an analogue or derivative thereof, and the animal is a pig, a chicken, a salmon, a cow or a human.

8. A method for the manufacture of a pharmaceutical composition which exhibits extended elaboration release suitable for parenteral administration according to any one of claims 1 to 7, comprising associating high-integrity liposomes which comprise at least one fully hydrogenated phosphatidylcholine stabile lipid which is not dicetylphosphate (DCP); and a lipid diluent selected from the group consisting of cholesterol and alphatocopherol, with a therapeutically effective amount of at least one peptide-like therapeutic agent, for use in a method of treating an animal by administering to said animal a therapeutically effective dose of said composition, and wherein said therapeutic agent is selected from the group consisting of antigens, hormones, immunomodulators, glycosylated carrier proteins and galactosylated carrier proteins, wherein said therapeutical agent is not a tetanus toxoid and wherein said liposome does not contain stearylamine, 6-(5-cholesten-3β-yloxy)hexyl 1-thio-β-L-fucopyranoside, 6-(5-cholesten-3β-yloxy)hexyl 1-thio-β-D-galactopyranoside, 6-(5-cholesten-3β-yloxy)hexyl 1-thio-α-D-mannopyranoside, 6-(5-cholesten-3-yloxy)hexyl 2-acetamido-2-deoxy-1-thio-β-D-galactopyranoside, 6-(5-cholesten-3β-yloxy)hexyl 6-amino-6-deoxy-1-thio-β-D-galactopyranoside, or 6-(5-cholesten-3β-yloxy)hexyl 6-amino-6-deoxy-1-thio-α-D-mannopyranoside.

9. A method for increasing milk production in dairy animals by administering to said animals a therapeutically effective amount of a somatotropin, or an analogue or derivative thereof, in the pharmaceutical composition of claim 4.

## Patentansprüche

1. Therapeutische pharmazeutische Zusammensetzung für die parenterale Verabreichung, die vervollkommnete Langzeit-Freisetzungseigenschaften bei parenteraler Verabreichung bei Lebewesen zeigt, wobei die Zusammensetzung in hohem Maße unversehrte Liposomen umfaßt, die a) wenigstens ein vollständig hydriertes stabiles Phosphatidylcholin-Lipid, das nicht Dicetylphosphat (DCP) ist; b) ein Lipidverdünnungsmittel, ausgewählt aus der Gruppe, die aus Cholesterol und α-Tocopherol besteht; und c) eine therapeutisch wirksame Menge von wenigstens einem peptidartigen therapeutischen Mittel das mit den Liposomen assoziiert ist, wobei das therapeutische Mittel aus der Gruppe ausgewählt ist, die aus Antigenen, Hormonen, Immunmodulatoren, glycosylierten Trägerproteinen und galactosylierten Trägerproteinen besteht, umfaßt, und worin das therapeutische Mittel kein Tetanustoxoid ist und worin das Liposom kein Stearylamin,
6-(5-Cholesten-3β-yloxy)hexyl-1-thio-β-L-fucopyranosid, 6-(5-Cholesten-3β-yloxy)hexyl-1-thio-β-D-galactopyranosid, 6-(5-Cholesten-3β-yloxy)hexyl-1-thio-α-D-Mannopyranosid, 6-(5-Cholesten-3-yloxy)hexyl-2-acetamido-2-deoxy-1-thio-β-D-galactopyranosid, 6-(5-Cholesten-3β-yloxy)hexyl-6-amino-6-deoxy-1-thio-β-D-galactopyranosid oder 6-(5-Cholesten-3β-yloxy)hexyl-6-amino-6-deoxy-1-thio-α-D-mannopyranosid enthält.

2. Zusammensetzung nach Anspruch 1, worin das stabile Lipid hydriertes Sojabohnen-Phosphatidylcholin oder Distearoyl-phosphatidylcholin ist.

3. Zusammensetzung nach Anspruch 1 oder 2, worin das Lipidverdünnungsmittel Cholesterol ist mit einem molaren Verhältnis Lipid:Cholesterol von 4:1 bis 1:1.

4. Zusammensetzung nach einem der Ansprüche 1 bis 3, worin das peptidartige therapeutische Mittel Galactose-Albumin, Calcitonin, ein Somatotropin, ein Interleuken oder ein Analog oder Derivat eines dieser Mittel ist.

5. Zusammensetzung nach Anspruch 4, worin das peptidartige therapeutische Mittel Interleukin-2 ist.

6. Zusammensetzung nach einem der Ansprüche 1 bis 3, worin das peptidartige therapeutische Mittel ein Peptid ist und die Liposomen als Adjuvans fungieren.

7. Zusammensetzung nach Anspruch 5, worin das peptidartige therapeutische Mittel Calcitonin oder ein Analog oder Derivat davon ist, und das Lebewesen ist ein Schwein, ein Huhn, ein Lachs, eine Kuh oder ein Mensch.

8. Verfahren zur Herstellung einer pharmazeutischen Zusammensetzung nach Anspruch 1 bis 7, die vervollkommnete Langzeit-Freisetzungseigenschaften zeigt, die für die parenterale Verabreichung geeignet ist, umfassend die Assoziierung von in hohem Maße unversehrten Liposomen, die wenigstens ein vollständig hydriertes stabiles Phosphatidylcholin-Lipid, das nicht Dicetylphosphat (DCP) ist, und ein Lipidverdünnungsmittel, ausgewählt aus der Gruppe, die aus Cholesterol und α-Tocopherol besteht, umfassen; mit einer therapeutisch wirksamen Menge von wenigstens einem peptidartigen therapeutischen Mittel, zur Verwendung in einem Verfahren zur Behandlung eines Lebewesens durch Verabreichung einer therapeutisch wirksamen Dosis der Zusammensetzung an das Lebewesen, und worin das therapeutische Mittel ausgewählt wird aus der Gruppe, die aus Antigenen, Hormonen, Immunmodulatoren, glycosylierten Trägerproteinen und galactosylierten Trägerproteinen besteht, worin das therapeutische Mittel kein Tetanustoxoid ist und worin das Liposom kein Stearylamin,
6-(5-Cholesten-3β-yloxy)hexyl-1-thio-β-L-fucopyranosid, 6-(5-Cholesten-3β-yloxy)hexyl-1-thio-β-D-galactopyranosid, 6-(5-Cholesten-3β-yloxy)hexyl-1-thio-α-D-Mannopyranosid, 6-(5-Cholesten-3-yloxy)hexyl-2-acetamido-2-deoxy-1-thio-β-D-galactopyranosid, 6-(5-Cholesten-3β-yloxy)hexyl-6-amino-6-deoxy-1-thio-β-D-galactopyranosid oder 6-(5-Cholesten-3β-yloxy)hexyl-6-amino-6-deoxy-1-thio-α-D-mannopyranosid enthält.

9. Verfahren zur Erhöhung der Milchleistung von Milchvieh durch Verabreichung einer therapeutisch wirksamen Menge eines Somatotropins oder eines Analogen oder Derivats davon in der pharmazeutischen Zusammensetzung von Anspruch 4 an die Tiere.

## Revendications

1. Composition pharmaceutique thérapeutique appropriée à l'administration parentérale et manifestant des caractéristiques de libération par émission prolongée, lors de l'administration parentérale à un animal, la composition comprenant des liposomes à haute intégrité qui comprennent a) au moins un lipide stable de type phosphatidylcholine totalement hydrogénée, qui n'est pas le dicétylphosphate (DCP); b) un diluant des lipides choisi parmi le cholestérol et l'α-tocophérol; et c) une quantité thérapeutiquement efficace d'au moins un agent thérapeutique de type peptide associé auxdits liposomes, dans laquelle ledit agent thérapeutique est choisi parmi des antigènes, des hormones, des immunomodulateurs, des protéines vectrices glycosylées et des protéines vectrices qalactosylées, dans laquelle ledit agent thérapeutique n'est pas un toxoïde tétanique et dans laquelle ledit liposome ne contient pas de stéarylamine, 6-(5-cholestén-3β-yloxy)hexyl-1-thio-β-L-fucopyrannoside, 6-(5-cholestén-3β-yloxy)hexyl-1-thio-β-D-galactopyrannoside, 6-(5-cholestén-3β-yloxy)hexyl-1-thio-α-D-mannopyrannoside, 6-(5-cholestén-3β-yloxy)hexyl-2-acétamido-2-désoxy-1-thio-β-D-galactopyrannoside, 6-(5-cholestén-3β-yloxy)hexyl-6-amino-6-désoxy-1-thio-β-D-galactopyrannoside ni 6-(5-cholestén-3β-yloxy)hexyl-6-amino-6-désoxy-1-thio-α-D-mannopyrannoside.

2. Composition selon la revendication 1, dans laquelle le lipide stable est la phosphatidylcholine ou la distéaroylphosphatidylcholine de soja hydrogénée.

3. Composition selon la revendication 1 ou 2, dans laquelle le diluant des lipides est le cholestérol en un rapport lipide:cholestérol allant de 4:1 à 1:1.

4. Composition selon l'une quelconque des revendications 1 à 3, dans laquelle l'agent thérapeutique de type peptide est la galactose-amine, la calcitonine, une somatotropine, une interleukine ou un analogue ou dérivé de l'une quelconque de celles-ci.

5. Composition selon la revendication 4, dans laquelle l'agent thérapeutique de type peptide est l'interleukine-2.

6. Composition selon l'une quelconque des revendications 1 à 3, dans laquelle l'agent thérapeutique de type peptide est un peptide et les liposomes fonctionnent en tant qu'adjuvant.

7. Composition selon la revendication 5, dans laquelle l'agent thérapeutique de type peptide est la calcitonine ou un analogue ou dérivé de celle-ci, et l'animal est le porc, le poulet, le saumon, la vache ou l'homme.

8. Procédé pour la préparation d'une composition pharmaceutique qui manifeste des caractéristiques une libération par émission prolongée, appropriée à l'administration parentérale, selon l'une quelconque des revendications 1 à 7, comprenant l'association de liposomes à haute intégrité qui comprennent au moins un lipide stable de type phosphatidylcholine totalement hydrogénée, qui n'est pas le dicétylphosphate (DCP), et un diluant des lipides choisi parmi le cholestérol et l'α-tocophérol, avec une quantité thérapeutiquement efficace d'au moins un agent thérapeutique de type peptide, destinée à être utilisée dans un procédé de traitement d'un animal par administration à cet animal d'une dose thérapeutiquement efficace de ladite composition, et dans lequel ledit agent thérapeutique est choisi parmi des antigènes, des hormones, des immunomodulateurs, des protéines vectrices glycosylées et des protéines vectrices galactosylées, dans lequel ledit agent thérapeutique n'est pas un toxoïde tétanique et dans lequel ledit liposome ne contient pas de stéarylamine, 6-(5-cholestén-3β-yloxy)hexyl-1-thio-β-L-fucopyrannoside, 6-(5-cholestén-3β-yloxy)hexyl-1-thio-β-D-galactopyrannoside, 6-(5-cholestén-3β-yloxy)hexyl-1-thio-α-D-mannopyrannoside, 6-(5-cholestén-3β-yloxy)hexyl-2-acétamido-2-désoxy-1-thio-β-D-galactopyrannoside, 6-(5-cholestén-3β-yloxy)hexyl-6-amino-6-désoxy-1-thio-β-D galactopyrannoside ni 6-(5-cholestén-3β-yloxy)hexyl-6-amino-6-désoxy-1-thio-α-D-mannopyrannoside.

9. Procédé d'augmentation de la production de lait chez des animaux producteurs de lait, par administration auxdits animaux d'une quantité thérapeutiquement efficace d'une somatotropine ou d'un analogue ou dérivé de celle-ci, dans la composition pharmaceutique de la revendication 4.
